# EUROPEAN PATENT SPECIFICATION

(11) **EP 1 328 300 B1**
(45) Date of publication and mention of the grant of the patent: **05.01.2005**
(21) Application number: 01976497.6
(22) Date of filing: 22.10.2001
(51) Int. Cl.: A61L 15/32, A61L 31/04

(54) **SELF-ADHESIVE HYDRATABLE MATRIX FOR TOPICAL THERAPEUTIC USE**
SELBSTKLEBENDE, HYDRATIERBARE MATRIX FÜR THERAPEUTISCHE ANWENDUNGEN
MATRICE HYDRATABLE AUTO-ADHESIVE A USAGE THERAPEUTIQUE TOPIQUE

(30) Priority: 23.10.2000 GB 0025882; 27.10.2000 WO PCT/GB00/04154; 03.05.2001 GB 0110881; 07.08.2001 GB 0119193; 07.08.2001 GB 0119196
(43) Date of publication of application: 23.07.2003
(73) Proprietor: Tissuemed Limited, Leeds LS14 6UF (GB)
(72) Inventor: BURNETT, Stuart, Wavertree, Liverpool LI5 9EU (GB); EDWARDSON, Peter, Andrew, David, Alwoodley, Leeds LS17 8EB (GB); MANDLEY, David, John, Selby, North Yorkshire YO8 4JQ (GB); VELADA, Jose, Bondgate, Selby, North Yorkshire YO8 3WB (GB)
(74) Representative: Jones, Stephen Anthony
(86) International application number: PCT/GB2001/004682
(87) International publication number: WO 2002/034304

(56) References cited:
- WO-A-00/10618
- WO-A-01/30405

## Description

This invention relates to a sheet, patch or film for topical application to internal and external surfaces of the body, for therapeutic purposes. In particular, the invention relates to a self-adhesive, biocompatible and hydratable polymeric matrix, which may be used for wound healing, joining, sealing and reinforcing weakened tissue, and for drug delivery.

The use of therapeutic materials in the form of a sheet, patch or film, for topical administration to either internal or external organs of the body, is well documented for a wide range of medical applications. A disadvantage of currently available products is that they require manipulation *in situ* in order to secure the product in place, particularly internally. This manipulation commonly involves either mechanical attachment (eg using sutures) or chemical reaction with underlying tissue - either with the aid of externally applied energy (eg light or radio frequency energy) or though interaction between two or more of the components.

In many instances the use of sutures is either not wholly effective (eg on the lung), or undesirable as their introduction gives rise to further areas of tissue weakness. The use of external energy for attachment can be both time-consuming and (in some cases) requires significant careful judgement on the part of the surgeon, to evaluate when sufficient energy has been delivered to effect attachment without damaging the underlying tissue. Also, chemical interaction between components creates a risk of unwanted polymerisation and possible side effects of the reaction itself.

WO 00/10618 discloses a flexible, hydratable and activatable sheet for therapeutic use, that bonds to tissue and retains its activity on bonding.

There has now been devised an improved form of sheet or the like, suitable for topical application, either internally or externally, that overcomes or substantially mitigates the above-mentioned or other disadvantages of the prior art.

According to the invention, there is provided a self adhesive, biocompatible and hydratable polymeric matrix in the form of a sheet, patch or film suitable for application to moist surfaces both inside and on the external surface of the body, the matrix comprising a naturally occurring or synthetic polymerisable and/or cross-linkable material that supports wound healing, and a synthetic polymer having bioadhesive properties, such properties enabling the matrix to adhere to underlying tissue by means of ionic and/or hydrogen bonding.

The sheet or the like according to the invention is advantageous primarily in that the bioadhesive properties of the synthetic polymer enable the sheet to be positioned securely without the use of sutures or other forms of external physical attachment. The sheet is thus easy to use and can be applied rapidly and precisely.

The sheet or the like according to the invention may comprise in addition a therapeutically effective agent, ie a drug or medicament, and may be used as a delivery vehicle for such an agent. However, other embodiments of the invention are not used in this way, and are free of drug or medicament.

An important feature of the sheet or the like according to the invention is that it is suitable for application to both internal and external surfaces of the body, ie it may be applied topically to the exterior of the body (eg to the skin) or to internal surfaces such as surfaces of internal organs exposed during surgical procedures.

The polymerisable and/or cross-linkable component of the matrix is preferably selected from the polysaccharides, polylactates, polyalcohols and proteins, and derivatives thereof. The polymerisable component of the matrix may be partially or fully cross-linked.

In certain preferred embodiments of the invention, the polymerisable and/or cross-linkable component of the matrix is a protein or proteinaceous material, in particular a protein or the like that can be cross-linked by the application of heat or electromagnetic energy (eg a structural protein such as collagen or a globular protein such as albumin).

A particularly preferred protein for use in the invention is albumin, particularly mammalian albumin such as porcine, bovine or human albumin.

In other preferred embodiments of the invention, the polymerisable and/or cross-linkable component of the matrix is a polysaccharide or a derivative thereof. Particular polysaccharides that may be mentioned include cellulose derivatives, particularly cellulose ethers and derivatives and salts thereof. Examples include carboxymethyl cellulose and salts thereof, hydroxypropylmethyl cellulose and hydroxyethylmethyl cellulose. Sodium carboxymethyl cellulose is one example of such a polymer.

The bioadhesive polymer component of the matrix may be any polymer with suitable bioadhesive properties, ie any polymer which confers on the matrix a sufficient degree of adhesion to the tissue to which it is applied. Such polymers typically contain chemical groups with a high ionic density, eg carboxyl, amide, hydroxyl, ether and ester groups, and the salts thereof, which interact cooperatively with tissue, through the formation of ionic and hydrogen bonds, dipole - dipole interactions and Van der Waals forces. Effective polymers are generally of high molecular weight since the degree of bioadhesion may be proportional to the number of these groups available. Typically, the molecular weight of the bioadhesive polymer will be in excess of about 100,000. The polymers are also generally linear, becoming physically entangled and having an amorphous distribution in solution. Preferably they should be able to be cross-linked to stabilise and strengthen the bioadhesive layer in the sheet, without compromising the bioadhesive properties. Examples of suitable polymers are poly(carboxylic acids) and their derivatives (ie polyanhydrides, polyesters), copolymers of carboxylic acids and their derivatives, polyalcohols and their derivatives.

A preferred group of bioadhesive polymers are polymers consisting of recurring structural units containing amide groups. Preferably, the recurring unit is, or contains a 1-ethylenepyrrolidin-2-one (vinylpyrrolidone) group. Homopolymers containing recurring vinylpyrrolidone groups are particularly preferred, ie poly(vinylpyrrolidone).

The bioadhesive polymer may alternatively be a copolymer, eg a copolymer of amide-containing units as described above and carboxylic acid ester-containing units, eg vinyl acetate units. One particular form of copolymer that may be suitable is thus poly(vinylpyrrolidone)/poly(vinylacetate) copolymer.

Other groups of polymers that may exhibit suitable bioadhesive properties include polymers which may also serve as the polymerisable and/or cross-linkable component of the matrix, such as cellulose derivatives, particularly cellulose ethers and derivatives and salts thereof. Examples include carboxymethyl cellulose and salts thereof, hydroxypropylmethyl cellulose and hydroxyethylmethyl cellulose. Sodium carboxymethyl cellulose is again one example of such a polymer.

Combinations of polymers of the kinds described above may be employed. One preferred example is a combination of a polymer of amide-containing units as described above and a cellulose derivative as described above. A particular combination is poly(vinylpyrrolidone) and a salt, eg the sodium salt, of carboxymethyl cellulose. In such a combination, the polymer of amide-containing units, eg poly(vinylpyrrolidone), is preferably present in a proportion of between 0.1 and 60 times that of the cellulose derivative, more preferably between 1 and 40 times. The polymer of amide-containing units is preferably the predominant component, ie it is present in a greater proportion than the cellulose derivative.

Where the matrix comprises both a polymer of amide-containing units, eg poly(vinylpyrrolidone), and a cellulose derivative, eg carboxymethyl cellulose, certain embodiments may further comprise another polymerisable and/or cross-linkable material, most preferably a protein or proteinaceous material, eg albumin.

Sufficiency of the degree of adhesion of the matrix to the tissue, by the bioadhesive polymer(s), can be quantitatively determined *in vitro*, for example by performing a peel strength test. This test is performed by allowing the matrix to adhere to a suitable substrate (secured in a fixed position), while the matrix itself is physically attached at a separate point to the load of a tensile testing apparatus, positioned so that prior to the test, the matrix is not under load. The load cell is moveable along an axis substantially perpendicular to that along which the substrate is positioned. The test involves movement of the load cell away from the substrate, at a constant predetermined rate, until the matrix detaches from the substrate. The output of the test is a quantitative measure of the peel fracture energy for that matrix - ie the cumulative amount of energy required to break the interaction between the matrix and the substrate to which it is adhered. A suitable cumulative peel fracture energy for the matrix according to the invention would be not less than 10,000 N/m, more preferably not less than 20,000 N/m.

The matrix preferably further comprises a plasticiser in order to ensure that the matrix has sufficient flexibility, even after polymerisation or cross-linking. Suitable plasticisers include polyalcohols, eg glycerol, sorbitol etc.

The matrix may also comprises a synthetic or biological structural polymer to confer strength and elasticity on the matrix. Suitable polymers include water-soluble thermoplastic polymers, in particular selected from the group consisting of poly(vinyl alcohol), poly(ethylene glycol), poly(acrylic acid), poly(acrylamide) and similar materials.

The bioadhesive polymer component of the matrix, eg poly(vinyl pyrrolidone), may also contribute to the structural properties of the matrix.

One or more surfactants, most preferably non-ionic surfactants, will generally be incorporated into the matrix, for instance to facilitate manufacture (eg to either prevent foaming, for production of closed structures, or to promote foaming, for the production of more mesh-like structures). Suitable surfactants include block copolymers of ethylene oxide and propylene oxide, such as those sold under the trade marks Pluronic® by BASF. In some instances, the proportion of surfactant incorporated into the matrix may be relatively low, eg less than 1%. In other embodiments, which have an open, mesh-like structure as described below, higher proportions of surfactants may be used, eg to create and stabilise a foam formed during manufacture.

The matrix in the form of a sheet, patch or film may be homogeneous or heterogeneous in composition, and may be of continuous or discontinuous structure. One or both major surfaces may have adhesive properties.

One group of preferred embodiments of the matrix according to the invention comprises the following proportions (percentages by weight) of the individual components:
a) polymerisable and/or cross-linkable material - from 2% to 80% by weight, more preferably 5% to 60%, and most preferably 10% to 30%;
b) bioadhesive polymer(s) - from 5% to 90% by weight, more preferably 20% to 80%, and most preferably 30% to 60%;
c) structural polymer - from 0.01% to 20% by weight, more preferably 1% to 15%, and more preferably 2% to 10%;
d) surfactant - from 0.001% to 10% more preferably 0.01% to 1%, and most preferably 0.01% to 0.1%;
e) plasticiser - from 1% to 70%, more preferably 10% to 60%, and most preferably 20% to 40%.

The matrix may contain between 2% and 60% water by weight, and most preferably between 5% and 30%. The matrix may be partially or totally hydrated with a suitable aqueous medium at or following implantation (eg a body fluid or saline solution).

Other embodiments, eg those prepared by lyophilization as described below, may be substantially free of water.

The matrix may be manufactured by combining solutions of the different components as follows (all amounts are percentage weight of the component in the respective solution prior to combination):
a) Solution A:
   i) polymerisable and/or cross-linkable material: 5 - 60%, more preferably 10 - 50%, and most preferably 20 - 40%.
   ii) structural polymer : 0.1 - 30%, more preferably 1 - 20%, and most preferably 3-10%.
   iii) surfactant : 0.001 - 5%, more preferably 0.01 - 1%, and most preferably 0.05- 0.5%.
   iv) plasticiser : 1 - 80%, more preferably 10 - 60%, and most preferably 15 - 35%.
b) Solution B:
   i) bioadhesive polymer(s) : 1 - 60%, more preferably 5 - 40%, and most preferably 10 - 30%.
   ii) plasticiser :1 - 60%, more preferably 5 - 40%, and most preferably 10 - 30%.

In a preferred embodiment, where one surface only, or a selected part thereof, is bioadhesive, the matrix may be prepared by casting Solution A into a suitable non-stick mould (eg of PTFE), and causing or allowing it to set through evaporation. Onto this is then cast Solution B, which is also caused or allowed to set. During this process, the second solution penetrates into, and chemically binds to, the matrix formed by the first solution, so that the final matrix is composed of a single sheet with concentration gradients of the various components.

Alternatively, the matrix may be prepared from a single solution comprising all the components, or by combination of multiple solutions to create multi-lamellar matrices (eg bioadhesive - polymeric matrix - bioadhesive).

The casting process used to achieve the desired thickness of the sheet may involve pouring, manual spreading or spraying of the component solutions.

When prepared as described above, the matrix according to the invention may be 20 - 1000 µm in thickness, and typically approximately 100 - 500 µm in thickness. Dimensionally, the patch or film may have a surface area of only a few square millimetres, extending to several tens of centimetres.

For some uses, it may be desirable to modify the stability of the sheet - such that the half-life of the product is extended (for use in reinforcement of weakened tissue) or reduced (for drug release). This modification of stability can be effected by controlling the extent of formation of covalent bonds between molecules in the matrix (eg formation of disulphide bonds between protein molecules). If an increase in patch stability is desired, the matrix can be pre-treated to induce the formation of intermolecular covalent bonds. The structural layer in particular may be partially or fully cross-linked.

Pre-treatment methods that can be used to modify the stability of the matrix are:
1) Heat: Heat may be used to partially or fully cross-link proteins and to drive off water from the bioadhesive component. Temperatures from 30-70°C will promote an unravelling of the polypeptide chains, which may reduce water solubility of the protein. Exposure of the matrix to temperatures between 70°C and 120°C will promote formation of covalent bonds between albumin molecules. This will increase the stability of the sheets, the degree of stability achieved being dependent on the precise time, and temperature of this pre-treatment.
2) Irradiation : Electromagnetic radiation (including visible and UV light, gamma irradiation and electron beam) can promote cross-linking of albumin molecules, and will polymerise the bioadhesive molecules. This is a potential method by which large sheets could be pre-treated in such a way as to increase their stability.
3) Chemical : There are a large variety of chemical cross-linking reagents which could potentially be used to induce formation of covalent bonds within the matrix, including chromophore dyes such as methylene blue.

In a particularly preferred embodiment the sheet or patch according to the invention is prepared from two or three separate layers, and the manufacture of the patch involves exposure to both heat and ionizing radiation. The structural layer containing a protein such as albumin is prepared first, and is partially or fully polymerised by exposure to heat for a given period of time. One or two additional bioadhesive layers are cast on top of the pre-formed structural layer - these are exposed to heat to evaporate off water, which may otherwise impede the bioadhesive nature of the final product. The completed patch is packaged and gamma-irradiated which both achieves inter- and intra-molecular polymerisation of the bioadhesive layer and sterilization of the patch. The former is necessary to optimise strength of the bioadhesive layer and create a tightly bound structure that will not delaminate, while the latter is necessary for implantation within the body cavity.

In an alternative embodiment, the patch may be presented in a lyophilized form, (to improve stability and enhance its absorptive capacity). The process of lyophilization (which involves freezing the patch at between -20°C and -70°C, and subsequently exposing the frozen patch to a vacuum to remove residual water) must take place after exposure of the patch to electromagnetic radiation. The residual water in the patch may be partially or totally removed during this process in order to achieve the required degree of absorption and elasticity.

In a further, related embodiment the patch may be presented in the form of a sponge, being mesh-like, and evidently open in structure, with only a minor proportion of the overall volume of the structure being occupied by solid material. In this case the patch is manufactured and exposed to Y-irradiation, and then swollen in water or a buffer to the required degree. Other aqueous solutions can be used to swell the patch in order to include the solute in the final product. The swollen patch is finally freeze-dried as above, to remove some or all of the water.

Embodiments of the invention having open, mesh-like structures may comprise the following proportions (percentages by weight) of the individual components:
a) bioadhesive, polymerisable and/or cross-linkable material - from 1% to 30% by weight, more preferably 5% to 30%, and most preferably 10% to 25%;
b) surfactant - from 0.01% to 20%, more preferably 0.1% to 15%, and most preferably 1% to 15%;
c) plasticiser - from 1% to 50%, more preferably 5% to 30%, and most preferably 10% to 25%.

Such embodiments may be manufactured by combining foamed solutions of the different components as follows (all amounts are percentage weight of the component in the respective solution prior to combination):
a) Solution A:
   i) bioadhesive, polymerisable and/or cross-linkable material: 5 - 35%, more preferably 10-30%, and most preferably 20-30%.
   ii) surfactant: 0.01-20%, more preferably 0.1-15%, and most preferably 1-15%.
b) Solution B:
   i) bioadhesive, polymerisable and/or cross-linkable material : 1-30%, more preferably 5-30%, and most preferably 10-25%.
   ii) plasticiser : 1-90%, more preferably 10-60%, and most preferably 10-50%.
   iii) surfactant: 0.01-20%, more preferably 0.1-15%, and most preferably 1-15%.

The solutions A and B may be agitated to form foams, typically rather viscous in nature, which are then mixed. The resulting mixture may have the form of a gel. Prior to freeze-drying (lyophilization), the mixture or gel is preferably cross-linked (most preferably by exposure to ionizing radiation) and swollen in water or a buffer solution.

Further exposure to ionizing radiation may follow lyophilization, in order to achieve sterilization.

Such embodiments of the invention may be provided on one surface with a continuous coating of a synthetic or naturally occurring polymeric material. Such a material may, for instance, be a water-soluble thermoplastic polymer, in particular selected from the group consisting of poly(vinyl alcohol), poly(ethylene glycol), poly(acrylic acid), poly(acrylamide) and similar materials.

According to a further aspect of the invention, there is therefore provided a process for the manufacture of a self-adhesive, biocompatible and hydratable polymeric matrix in the form of a sheet, patch or film suitable for application to moist surfaces both inside and on the external surface of the body, which process comprises forming a foamed solution of a naturally occurring or synthetic polymerisable and/or cross-linkable material that supports wound healing, and a synthetic polymer having bioadhesive properties, and subjecting said foamed solution to freeze-drying.

The process may comprise foaming a solution containing all the components of the matrix. Alternatively, the process may involve forming a first solution of the naturally occurring or synthetic polymerisable and/or cross-linkable material, and a second solution of the synthetic polymer having bioadhesive properties, foaming the first solution and the second solution, and then mixing the first and second solutions.

Manufacture of the matrix according to the invention is preferably carried out at reduced pH, preferably at a pH of less than 4.0, more preferably less than 3.0, eg about pH 2.0. Where the components of the matrix contain carboxy groups, eg where the matrix includes carboxymethyl cellulose, it is believed that the reduced pH increases the number of protonated carboxyl groups present. This in turn increases the hydrogen bonding capacity of the carboxyl groups (hydrogen bonding occurring for instance between the carboxyl groups of the carboxymethyl cellulose and carbonyl groups present in a polymer of recurring amide-containing units). This increased hydrogen bonding strengthens the polymer network of the gel, which in turn limits the degree of swelling that the gel undergoes. This provides benefits for the product in terms of ease of manufacturing, improved handling and improved structural integrity following implantation. Additionally, manufacture and swelling at low pH may provide the product with advantageous efficacy properties such as a localised physiological environment optimised for triggering the blood clotting cascade, so leading to rapid haemostasis.

Where the manufacturing process involves the use of reduced pH, the solutions of the various components may be made up in a low pH buffer, rather than in water, and/or a reduced pH buffer may be used to swell the gel produced in the course of manufacture.

Embodiments of the invention prepared by freeze-drying of foamed solutions may have thicknesses of 0.1 to 10mm or more, typically 0.5 to 8mm, more commonly 0.5 to 5mm.

The sheet, patch or film according to the invention is particularly suitable for surgical applications in the following areas:
Thoracic / cardiovascular
General surgery
ENT
Urology
Oral / maxillofacial
Orthopaedic
Neurological
Gastroenterology
Ophthalmology
Gynaecology / obstetrics

Possible uses are described in more detail below.

### Wound healing

The biodegradable nature of the sheet means that it may support and promote wound healing both during internal and topical procedures. Once the sheet begins to degrade fibroblasts will move in and begin to deposit components of the extracellular matrix. The sheet therefore can be used as an internal or external dressing. In addition, factors such as growth factors and cAMP that are known to promote the proliferation of skin cells may be added to the sheet to assist in the healing process. The sheet may act as a barrier to moisture and infectious agents, and thus be useful particularly in the treatment of burns.

### Skin closure

The sheet may be applied topically to promote wound closure (as an alternative to sutures). This may have beneficial effects in that it may reduce scarring, and the sheet may thus be useful for cosmetic purposes during minor surgery (eg in Accident and Emergency Departments). The self-adhesive properties of the patch would make it easy to apply quickly.

### Hernia repair

A 'stabilised' form of the sheet may be used to provide reinforcement in hernia repair procedures. The self-adhesive attachment overcomes the potential issues faced by conventional surgical reinforcing mesh products, which require suturing or stapling in an already weakened area. The patch for such a procedure may be engineered to have short or long term durability, depending on the degree of tissue repair required.

### Anastomosis

The self-adhesive patch formulation described here provides a means of rapid sealing of, and prevention of leaks in, joined tubular structures such as blood vessels, and vascular and bladder grafts, and the Gl tract. The ability of the patch to support tissue repair may be of particular value here if used in nerve repair.

### Sealing large areas of tissue

The good sealing and dry / wet handling properties of the patch, combined with its self-adhesive properties and ability to be manufactured to cover a large surface area, mean that it may be of particular use in sealing resected tissue surfaces - in particular those where diffuse bleeding is an issue (eg the liver). The patch also provides an ideal support matrix for tissue repair at such sites. This could also be applicable to limiting leakage of cerebro-spinal fluid following neurological surgery.

### Sealing air leaks

In addition to the patch properties described above, the high tensile strength and good inherent elasticity of the patch, make it particularly suitable for sealing air leaks in the lung, particularly following lung resection. Again, after effecting a seal, the patch provides an ideal support matrix for tissue repair at such sites.

### Therapeutic agent administration

Drugs and other therapeutic agents (including biologically active agents such as growth factors, and even cellular components) may be added to the solution(s) used to form the patch product, or covalently linked to components prior to their use in patch formation. Once the patch is in place, following application to the desired site, the drug will be slowly released from the patch, either by diffusion out of the sheet, or by engineering the sheet so that as it degrades over time the drug is released. The rate of release can be controlled by appropriated design of the matrix. The patch thus provides a means of delivering a known amount of drug either systemically or to a precise locus. The drug may be directly bound to the protein, sandwiched between layers of the patch or simply dispersed in the matrix.

### Prevention of Post-Surgical Adhesions

Post-surgical adhesion, the formation of undesired connective tissue between adjacent tissues, is a serious problem which can give rise to major post-surgical complications. It is a particular problem in bowel surgery where it can cause, for instance, twisting of the bowel which may then necessitate further surgical intervention. It has been found that the application of sheet material having self-adhesive properties in accordance with the invention to tissues exposed in a surgical procedure can be effective in preventing post-surgical adhesions between that tissue and neighbouring tissues.

Thus, according to another aspect of the invention there is provided a method for the prevention or inhibition of post-surgical adhesion, which method comprises applying to one or more tissues exposed in a surgical procedure a hydratable polymeric matrix in the form of a sheet, patch or film, the matrix comprising a naturally occurring or synthetic polymerisable and/or cross-linkable material and a synthetic polymer having bioadhesive properties.

A related aspect of the invention provides the use of a hydratable polymeric matrix in the form of a sheet, patch or film, the matrix comprising a naturally occurring or synthetic polymerisable and/or cross-linkable material and a synthetic polymer having bioadhesive properties, in the manufacture of a composition for the prevention or inhibition of post-surgical adhesion.

The invention will now be described in greater detail, by way of illustration only, with reference to the following Examples.

### Example 1

### Bilayer Hydrogel Patch

A solution in water comprising 28.6% porcine albumin, 17% glycerol, 5% PVA and 0.1% Pluronic 25R2 was cast onto a PTFE-coated flat surface and spread to a thickness of 70µm. This solution was heated to 100 °C for 10 minutes and allowed to cool.

A second solution in water, comprising 9.6% PVP K-90D, 9.7% CMC90 and 9.5% glycerol was similarly cast on top of the previously formed layer, to a thickness of 600 µm. The matrix was heated further to 100 °C for 10 minutes, and again allowed to cool.

The resulting bilayer hydrogel patch (approximately 140 µm thickness) was cut to size and sealed inside foil pouches. The individual patches were subsequently γ-irradiated. In a study to evaluate the utility of these patches for sealing sutured anastomses against blood loss, a self-adhesive patch of size 2cm x 2cm was applied over end-to-end conventionally sutured carotid artery anastomoses in each of six rabbits. The animals were recovered and maintained for 21 days. Acute blood loss from anastomoses treated with the patches (mean = 0.14g) was significantly lower (p=0.011) than that of sutured alone controls (mean = 2.7g). Subsequent healing of the treated anastomoses was comparable to that of sutured only controls, demonstrating that the patch supported tissue repair and natural healing processes.

### Example 2

### Trilayer Hydrogel Patch

A solution in water comprising 28.3% porcine albumin, 18.1% glycerol, 5% PVA and 0.1% Pluronic 25R2, was cast onto a PTFE-coated flat surface and spread to a thickness of 140µm. This solution was heated to 100 °C for 10 minutes and allowed to cool.

A second solution in water, comprising 23.2% PVP K-90D and 12.6% glycerol, was similarly cast on top of the previously formed layer, also to a thickness of 140 µm. The matrix was heated further to 100 °C for 10 minutes, and again allowed to cool.

A third solution in water comprising 17.8% PVP K-90D, 9.7% glycerol and 0.01% CMC AF3285, was cast on to the second layer, and spread to a thickness of 600µm. The entire matrix was then heated to 100 °C for 15 minutes, and allowed to cool.

The resulting trilayer hydrogel patch (approximately 330 µm thickness) was cut to size and sealed inside foil pouches. The individual patches were subsequently γ-irradiated.

Separately a viscous hydrogel was formed, comprising PVP K90-D (9.8% w/w)), CMC-90 (9.4% w/w) and glycerol (9.5% w/w). This was filled into a syringe, sealed inside a foil pouch and γ-irradiated.

A preclinical study in rabbits was undertaken to evaluate the utility of the gel + self-adhesive patch for sealing tissue defects against blood loss. A 6 mm punch biopsy was performed in the liver of each of six animals. The gel was first applied to the wound to fill the injury, and then a 4 cm² self-adhesive patch was placed over the wound site, and allowed to adhere. The animals were recovered and maintained for 16 days. Acute blood loss from wound sites treated with the patches (mean = 1.3g) was significantly lower (p=0.033) than that of untreated controls (mean = 13.0g). Subsequent healing of patch + gel treated wounds was comparable to that of controls in which the wound was stabilised with the cellulose-based haemostat patch sold under the trade name Surgicel, demonstrating that the patch supported tissue repair.

### Example 3

### Bilayer Hydrogel Patch

A solution in water comprising 28.3% porcine albumin, 18.1% glycerol, 5% PVA and 0.1% Pluronic 25R2, was cast onto a PTFE-coated flat surface and spread to a thickness of 210 µm. This solution was heated to 90 °C for 5 minutes and allowed to cool.

A second solution in water, comprising 13.2% PVP K-90D, 13.7% glycerol and 13.6% CMC-90, was similarly cast on top of the previously formed layer, to a thickness of 1200 µm. The matrix was heated further to 100 °C for 20 minutes, and again allowed to cool.

The resulting bilayer hydrogel patch (approximately 360 µm thickness) was cut to size and sealed inside foil pouches. The individual patches were subsequently γ-irradiated.

Separately a viscous hydrogel was formed, comprising PVP K90-D (9.8% w/w)), CMC-90 (9.4% w/w) and glycerol (9.5% w/w). This was filled into a syringe, sealed inside a foil pouch and γ-irradiated.

An acute preclinical study in rabbits was undertaken to evaluate the utility of the gel + self-adhesive patch for sealing tissue injury in the lung against air leak. A circular injury (approximately 10mm in diameter and 5mm deep) was made to a lung in each of five animals. The gel was first applied to the injury to fill the defect, and then a 4 cm² self-adhesive patch was placed over the wound site, and allowed to adhere to the underlying tissue. Each treated wound was observed for a maximum of 10 minutes. In all cases, air leak from the treated injury was markedly lower than in the untreated injury and in 3 cases, there was no air leak observed from the treated injuries.

### Example 4

### Freeze-Dried Bilayer Patch

A solution in water comprising 28.6% porcine albumin, 17% glycerol, 5% PVA and 0.1% Pluronic 25R2 was cast onto a PTFE-coated flat surface and spread to a thickness of 70µm. This solution was heated to 100 °C for 10 minutes and allowed to cool.

A second solution in water, comprising 9.6% PVP K-90D, 9.7% CMC90 and 9.5% glycerol was similarly cast on top of the previously formed layer, to a thickness of 600 µm. The matrix was heated further to 100 °C for 10 minutes, and again allowed to cool.

The resulting bilayer patch was γ-irradiated (25 - 40 kGy) to achieve cross-linking. The patch was then frozen at -30°C for 12 hours. The samples were then freeze-dried at -20°C for 48 hours followed by 12 hours at 0°C and finally 24 hours at 25°C. The dried samples were terminally sterilised by γ-irradiation (25 - 40 kGy).

### Example 5

### Freeze-Dried Monolayer Patch

Solution A: Pluronic F-68 (2.5 g) and Pluronic F-127 (2.5 g) copolymers were added slowly to 68.5 g of water for injection stirred at 300 rpm. The same level of stirring was maintained until the copolymers were dissolved. The speed of the stirrer was increased to 2500 rpm to form a foam that was typically 4 times the volume of the original solution. Poly(vinyl pyrrolidone) K-90D (26.5 g) was then added to the vortex of the foam to avoid formation of lumps. The foamy viscous solution was left to settle for 12 hours before being used.

Solution B: Glycerol (12.6 g) was mixed with 69.9 g of water for injection using a rotor stirrer set at 300 rpm. Pluronic F-68 (2.5 g) and Pluronic F-127 (2.5 g) copolymers were added slowly to the water/glycerol solution and the same level of stirring was maintained until the copolymers were dissolved. The speed of the stirrer was increased to 2500 rpm to form a foam that was typically 4 times the volume of the original solution. Sodium carboxymethyl cellulose Blanose 7LF (12.6 g) was then added to the vortex of the foam to avoid formation of lumps. The foamy viscous solution was left to settle for 12 hours before being used.

63 g of Solution B were thoroughly mixed with 37 g of solution A. The resultant gel was put into suitable moulds and γ-irradiated (25 - 40 kGy). This process produced a crosslinked gel that was then swollen in water for injection or pH 2 buffer (i.e. citric acid 0.03M/NaCl 0.061M/HCl 0.0082M) for 72 hours.

The swollen gels were transferred to trays and frozen at -30°C for 12 hours. The samples were then freeze-dried at -20°C for 48 hours followed by 12 hours at 0°C and finally 24 hours at 25°C. The dried samples were terminally sterilised by γ-irradiation (25 - 40 kGy).

### Example 6

### Freeze-Dried Monolayer Patch

Solution A: Pluronic F-68 (2.5 g) and Pluronic F-127 (2.5 g) copolymers were added slowly to 68.5 g of pH 2 buffer (i.e. citric acid 0.03M/NaCl 0.061M/HCl 0.0082M) stirred at 300 rpm. The same level of stirring was maintained until the copolymers were dissolved. The speed of the stirrer was increased to 2500 rpm to form a foam that was typically 4 times the volume of the original solution. Poly(vinyl pyrrolidone) K-90D (26.5 g) was then added to the vortex of the foam to avoid formation of lumps. The foamy viscous solution was left to settle for 12 hours before being used.

Solution B: Glycerol (12.6 g) was mixed with 69.9 g of pH 2 buffer (i.e. citric acid 0.03M/NaCl 0.061M/HCl 0.0082M) using a rotor stirrer set at 300 rpm. Pluronic F-68 (2.5 g) and Pluronic F-127 (2.5 g) copolymers were added slowly to the aqueous solution and the same level of stirring was maintained until the copolymers were dissolved. The speed of the stirrer was increased to 2500 rpm to form a foam that was typically 4 times the volume of the original solution. Sodium carboxymethyl cellulose Blanose 7LF (12.6 g) was then added to the vortex of the foam to avoid formation of lumps. The foamy viscous solution was left to settle for 12 hours before being used.

63 g of Solution B were thoroughly mixed with 37 g of solution A. The resultant gel was put into suitable moulds and γ-irradiated (25 - 40 kGy). This process produced a crosslinked gel that was then swollen in water for injection or pH 2 buffer (i.e. citric acid 0.03M/NaCl 0.061M/HCl 0.0082M) for 72 hours.

The swollen gels were transferred to trays and frozen at -30°C for 12 hours. The samples were then freeze-dried at -20°C for 48 hours followed by 12 hours at 0°C and finally 24 hours at 25°C. The dried samples were terminally sterilised by γ-irradiation (25 - 40 kGy).

### Example 7

### Freeze-Dried Monolayer Patch

Solution A: Poly(vinyl alcohol) 80% hydrolysed (2.8 g) was added slowly to 70.4 g of water for injection stirred at 300 rpm, the same level of stirring was maintained until the polymer was dissolved. The speed of the stirrer was increased to 2500 rpm to form a foam that was typically 4 times the volume of the original solution. Poly(vinyl pyrrolidone) K-90D (26.8 g) was then added to the vortex of the foam to avoid formation of lumps. The foamy viscous solution was left to settle for 12 hours before being used.

Solution B: Glycerol (13 g) was mixed with 72.5 g of water for injection using a rotor stirrer set at 300 rpm. Poly(vinyl alcohol) 80% hydrolysed (1.5 g) was added slowly to the aqueous solution and the same level of stirring was maintained until the polymer was dissolved. The speed of the stirrer was increased to 2500 rpm to form a foam that was typically 4 times the volume of the original solution. Sodium carboxymethyl cellulose Blanose 7LF (13 g) was then added to the vortex of the foam to avoid formation of lumps. The foamy viscous solution was left to settle for 12 hours before being used.

62.6 g of Solution B were thoroughly mixed with 37.4 g of solution A. The resultant gel was put into suitable moulds and γ-irradiated (25 - 40 kGy). This process produced a crosslinked gel that was then swollen in water for injection or pH 2 buffer (i.e. citric acid 0.03M/NaCl 0.061M/HCl 0.0082M) for 72 hours.

The swollen gels were transferred to trays and frozen at -30°C for 12 hours. The samples were then freeze-dried at -20°C for 48 hours followed by 12 hours at 0°C and finally 24 hours at 25°C. The dried samples were terminally sterilised by γ-irradiation (25 - 40 kGy).

### Example 8

### Coated Freeze-Dried Patch

12.5 g of poly(vinyl alcohol) 28-99 (Mw = 145000, 99 - 99.8% hydrolysed) were dissolved in 100 ml of water for injection at 95°C. The solution was allowed to cool down to room temperature.

5 x 5 cm samples of freeze-dried material prepared in Example 5 were uniformly coated with 0.5 ml of the above PVA 28-99 solution. The coated patches were put in a freezer set at -20°C for 12 hours. The samples were thawed at room temperature under vacuum and frozen at -20°C for another 12 hours. Finally, the patches were thawed to room temperature under vacuum, packed in aluminium foil pouches and terminally sterilised by γ-irradiation (25 - 40 kGy).

## Claims

1. A self-adhesive, biocompatible and hydratable polymeric matrix in the form of a sheet, patch or film suitable for application to moist surfaces both inside and on the external surface of the body, the matrix comprising a naturally occurring or synthetic polymerisable and/or cross-linkable material that supports wound healing, and a synthetic polymer having bioadhesive properties, such properties enabling the matrix to adhere to underlying tissue by means of ionic and/or hydrogen bonding.

2. A matrix as claimed in Claim 1, which further comprises a drug or medicament, the matrix serving as a delivery vehicle for the drug or medicament.

3. A matrix as claimed in Claim 1, which is free of drug or medicament.

4. A matrix as claimed in any preceding claim, wherein the polymerisable and/or cross-linkable component of the matrix is selected from the polysaccharides, polylactates, polyalcohols and proteins.

5. A matrix as claimed in Claim 4, wherein the polymerisable and/or cross-linkable component of the matrix is a protein or proteinaceous material that can be cross-linked by the application of heat or electromagnetic energy.

6. A matrix as claimed in Claim 5, wherein the matrix comprises albumin.

7. A matrix as claimed in Claim 6, wherein the albumin is mammalian albumin such as porcine, bovine or human albumin.

8. A matrix as claimed in any one of Claims 1 to 3, wherein the polymerisable and/or cross-linkable component of the matrix is a polysaccharide or a derivative thereof.

9. A matrix as claimed in Claim 8, wherein the polysaccharide is a cellulose derivative.

10. A matrix as claimed in Claim 9, wherein the cellulose derivative is a cellulose ether or a derivative or a salt thereof.

11. A matrix as claimed in claim 9, wherein the cellulose derivative is sodium carboxymethyl cellulose.

12. A matrix as claimed in any preceding claim, wherein the bioadhesive polymer component of the matrix contains carboxyl, amide, hydroxyl, ether or ester groups.

13. A matrix as claimed in Claim 12, wherein the bioadhesive polymer is selected from the group consisting of poly(carboxylic acids) and their derivatives, copolymers of carboxylic acids and their derivatives, and polyalcohols and their derivatives.

14. A matrix as claimed in Claim 12, wherein the bioadhesive polymer consists of recurring structural units containing amide groups.

15. A matrix as claimed in Claim 14, wherein the recurring unit is, or contains, a 1-ethylenepyrrolidin-2-one (vinylpyrrolidone) group.

16. A matrix as claimed in Claim 14, wherein the polymer is poly(vinylpyrrolidone).

17. A matrix as claimed in Claim 12, wherein the bioadhesive polymer is a copolymer of amide-containing units and carboxylic acid ester-containing units.

18. A matrix as claimed in Claim 17, wherein the copolymer is poly(vinylpyrrolidone)/poly(vinylacetate) copolymer.

19. A matrix as claimed in Claim 1, wherein the polymer having bioadhesive properties is a cellulose derivative.

20. A matrix as claimed in Claim 19, wherein the cellulose derivative is a cellulose ether or a derivative or a salt thereof.

21. A matrix as claimed in Claim 20, wherein the cellulose derivative is sodium carboxymethyl cellulose.

22. A matrix as claimed in Claim 1, which comprises a combination of a polymer of amide-containing units and a cellulose derivative.

23. A matrix as claimed in Claim 22, which comprises poly(vinylpyrrolidone) and a carboxymethyl cellulose derivative or salt thereof.

24. A matrix as claimed in Claim 23, which comprises sodium carboxymethyl cellulose.

25. A matrix as claimed in Claim 22, wherein the polymer of amide-containing units is present in a proportion of between 0.1 and 60 times that of the cellulose derivative.

26. A matrix as claimed in Claim 25, wherein the polymer of amide-containing units is present in a greater proportion than the cellulose derivative.

27. A matrix as claimed in any preceding claim, which has a peel fracture energy of not less than 10,000 N/m.

28. A matrix as claimed in any preceding claim, which further comprises a plasticiser.

29. A matrix as claimed in Claim 28, wherein the plasticiser is a polyalcohol.

30. A matrix as claimed in Claim 29, wherein the plasticiser is glycerol.

31. A matrix as claimed in any preceding claim, wherein the matrix further comprises a synthetic or biological structural polymer to confer strength and elasticity on the matrix.

32. A matrix as claimed in Claim 31, wherein the structural polymer is selected from the group consisting of poly(vinyl alcohol), poly(ethylene glycol), poly(acrylic acid), poly(acrylamide) and similar materials.

33. A matrix as claimed in any preceding claim, which further comprises one or more surfactants.

34. A matrix as claimed in Claim 33, which comprises a surfactant in the form of a copolymer of ethylene oxide and propylene oxide.

35. A matrix as claimed in Claim 1, which comprises:
a) polymerisable and/or cross-linkable material - from 2% to 80% by weight, more preferably 5% to 60%, and most preferably 10% to 30%;
b) bioadhesive polymer(s) - from 5% to 90% by weight, more preferably 20% to 80%, and most preferably 30% to 60%;
c) structural polymer - from 0.01% to 20% by weight, more preferably 1% to 15%, and most preferably 2% to 10%;
d) surfactant - from 0.001% to 10% more preferably 0.01% to 1%, and most preferably 0.01% to 0.1%;
e) plasticiser - from 1% to 70%, more preferably 10% to 60%, and most preferably 20% to 40%.

36. A matrix as claimed in any preceding claim, which contains between 2% and 60% water by weight, and preferably between 5% and 30%.

37. A process for the manufacture of a matrix as claimed in any preceding claim, which process comprises forming solutions of the following compositions:
a) Solution A:
i) polymerisable and/or cross-linkable material: 5 - 60%, more preferably 10 - 50%, and most preferably 20 - 40%.
ii) structural polymer : 0.1 - 30%, more preferably 1 - 20%, and most preferably 3-10%.
iii) surfactant : 0.001 - 5%, more preferably 0.01 -1%, and most preferably 0.05- 0.5%.
iv) plasticiser : 1 - 80%, more preferably 10 - 60%, and most preferably 15 - 35%.
b) Solution B:
i) bioadhesive polymer(s) : 1 - 60%, more preferably 5 - 40%, and most preferably 10 - 30%.
ii) plasticiser :1 - 60%, more preferably 5 - 40%, and most preferably 10 - 30%.
combining Solution A with Solution B, and causing or allowing the combined solutions to set.

38. A process as claimed in Claim 37, wherein said combining comprises casting Solution A in a mould and causing or allowing it to set by evaporation to form a first layer, and casting Solution B onto the first layer.

39. A matrix as claimed in claim 1, which has the form of a sponge, being mesh-like, and evidently open in structure, with only a minor proportion of the overall volume of the structure being occupied by solid material.

40. A matrix as claimed in Claim 39, which comprises:
a) bioadhesive, polymerisable and/or cross-linkable material - from 1% to 30% by weight, more preferably 5% to 30%, and most preferably 10% to 25%;
b) surfactant - from 0.01% to 20%, more preferably 0.1% to 15%, and most preferably 1% to 15%;
c) plasticiser - from 1% to 50%, more preferably 5% to 30%, and most preferably 10% to 25%.

41. A matrix as claimed in Claim 39 or Claim 40, one surface of which has a continuous coating of a synthetic or naturally occurring polymeric material.

42. A process for the manufacture of a matrix as claimed in any one of Claims 39 to 41, which process comprises forming solutions of the following compositions:
a) Solution A:
i) bioadhesive, polymerisable and/or cross-linkable material: 5 - 35%, more preferably 10-30%, and most preferably 20-30%.
ii) surfactant: 0.01-20%, more preferably 0.1-15%, and most preferably 1-15%.
b) Solution B:
i) bioadhesive, polymerisable and/or cross-linkable material: 1-30%, more preferably 5-30%, and most preferably 10-25%.
ii) plasticiser : 1-90%, more preferably 10-60%, and most preferably 10-50%.
iii) surfactant: 0.01-20%, more preferably 0.1-15%, and most preferably 1-15%. agitating the solutions A and B to form foams, mixing the solutions A and B to form a gel, and freeze-drying the gel.

43. A process as claimed in Claim 42, which further comprises cross-linking of the gel.

44. A process as claimed in claim 43, wherein the cross-linking is carried out by exposing the gel to ionizing radiation.

45. A process as claimed in any one of claims 42 to 44, which further comprises swelling of the gel.

46. A process for the manufacture of a self-adhesive, biocompatible and hydratable polymeric matrix in the form of a sheet, patch or film suitable for application to moist surfaces both inside and on the external surface of the body, which process comprises forming a foamed solution of a naturally occurring or synthetic polymerisable and/or cross-linkable material that supports wound healing, and a synthetic polymer having bioadhesive properties, and subjecting said foamed solution to freeze-drying.

47. A process as claimed in Claim 46, which comprises foaming a solution containing all the components of the matrix.

48. A process as claimed in Claim 46, which comprises forming a first solution of the naturally occurring or synthetic polymerisable and/or cross-linkable material, and a second solution of the synthetic polymer having bioadhesive properties, foaming the first solution and the second solution, and then mixing the first and second solutions.

49. A process as claimed in any one of Claims 42 to 48, which is carried out at reduced pH.

50. A process as claimed in Claim 49, which is carried out at a pH of less than 4.0.

51. A process as claimed in Claim 49, which is carried out at a pH of less than 3.0.

52. A process as claimed in any one of Claims 49 to 51, which involves forming one or more solutions in a low pH buffer.

53. A process as claimed in any one of Claims 49 to 52, which involves swelling of the gel in a low pH buffer.

54. A matrix as claimed in Claim 1, which has a thickness of 20 -1000 µm.

55. A matrix as claimed in Claim 39, which has a thickness of 0.1 to 10mm.

56. The use of a matrix as claimed in any one of Claims 1 to 36 or 39 to 41 in the manufacture of a composition for the prevention or inhibition of post-surgical adhesion.

## Patentansprüche

1. Eine selbstklebende, umweltverträgliche und hydratisierbare polymere Gewebeschicht in Form eines Blattes, eines Pflasters oder einer Membran, die für die Anwendung auf feuchten Oberflächen, sowohl innen als auch auf der äußeren Oberfläche des Körpers, geeignet ist, wobei die Gewebeschicht einen natürlich vorkommenden oder synthetisch polymerisierbaren und/oder vernetzbaren Stoff, der die Wundheilung fördert, und ein synthetisches Polymer, das bioadhesive Eigenschaften, wobei solche Eigenschaften der Gewebeschicht ermöglichen, mittels einer ionischer Verbindung und/oder einer Wasserstoffverbindung an dem darunter liegenden Gewebe anzuhaften, besitzt, umfasst.

2. Eine Gewebeschicht nach Anspruch 1, die außerdem ein Arznei- oder Heilmittel umfasst, wobei die Gewebeschicht als Übertragungsmedium für das Arznei- oder Heilmittel dient.

3. Eine Gewebeschicht nach Anspruch 1, die frei von Arznei- oder Heilmitteln ist.

4. Eine Gewebeschicht nach einem der vorhergehenden Ansprüche, wobei die polymerisierbare und/oder vernetzbare Komponente der Gewebeschicht von Polysacchariden, Polylaktaten, Polyalkoholen und Proteinen ausgewählt ist.

5. Eine Gewebeschicht nach Anspruch 4, wobei die polymerisierbare und/oder vernetzbare Komponente der Gewebeschicht ein Protein oder ein proteinhaltiger Stoff, der durch die Anwendung von Hitze oder elektromagnetischer Energie vernetzt werden kann, ist.

6. Eine Gewebeschicht nach Anspruch 5, wobei die Gewebeschicht Eiweiß umfasst.

7. Eine Gewebeschicht nach Anspruch 6, wobei das Eiweiß ein Säugetiereiweiß, wie Schweine- oder Rindereiweiß oder menschliches Eiweiß, ist.

8. Eine Gewebeschicht nach einem der vorhergehenden Ansprüche 1 bis 3, wobei die polymerisierbare und/oder vernetzbare Komponente der Gewebeschicht ein Polysaccharid oder ein Derivat davon ist.

9. Eine Gewebeschicht nach Anspruch 8, wobei das Polysaccharid ein Zellulose-Derivat ist.

10. Eine Gewebeschicht nach Anspruch 9, wobei das Zellulose-Derivat ein Zellulose-Äther oder ein Derivat oder ein Salz davon ist.

11. Eine Gewebeschicht nach Anspruch 9, wobei das Zellulose-Derivat eine Natriumcarboxylmethyl-Zellulose ist.

12. Eine Gewebeschicht nach einem der vorhergehenden Ansprüche, wobei die bioadhesive polymere Komponente der Gewebeschicht Carboxyl-, Amid-, Hydroxyl-, Äther- oder Estergruppen enthält.

13. Eine Gewebeschicht nach Anspruch 12, wobei das bioadhesive Polymer aus der Gruppe, bestehend aus Polycarbonsäuren und deren Derivate, Copolymere von Carbonsäuren und deren Derivate und Polyalkohole und deren Derivate, ausgewählt ist.

14. Eine Gewebeschicht nach Anspruch 12, wobei das bioadhesive Polymer aus einer periodischen Struktureinheit, die Amidgruppen enthält, besteht.

15. Eine Gewebeschicht nach Anspruch 14, wobei die periodische Einheit eine 1-Äthylenpyrrolidin-2-eins(Vinylpyrrolidon-) Gruppe ist oder beinhaltet.

16. Eine Gewebeschicht nach Anspruch 14, wobei das Polymer ein Polyvinylpyrrolidon ist.

17. Eine Gewebeschicht nach Anspruch 12, wobei das bioadhesive Polymer ein Copolymer aus Amid beinhaltenden Einheiten und Carbonsäurenester beinhaltenden Einheiten ist.

18. Eine Gewebeschicht nach Anspruch 17, wobei das Copolymer ein Polyvinylpyrrolidon-/ Polyvinylazetat-Polymer ist.

19. Eine Gewebeschicht nach Anspruch 1, wobei das Polymer, das bioadhesive Eigenschaften besitzt, ein Zellulose-Derivat ist.

20. Eine Gewebeschicht nach Anspruch 19, wobei das Zellulose-Derivat ein Zellulose-Äther oder ein Derivat oder ein Salz davon ist.

21. Eine Gewebeschicht nach Anspruch 20, wobei das Zellulose-Derivat eine Natriumcarboxylmethyl-Zellulose ist.

22. Eine Gewebeschicht nach Anspruch 1, die eine Kombination aus einem Polymer von Amid beinhaltenden Einheiten und einem Zellulose-Derivat umfasst.

23. Eine Gewebeschicht nach Anspruch 22, die ein Polyvinylpyrrolidon und ein Carboxylmethyl-Zellulose-Derivat oder Salz davon umfasst.

24. Eine Gewebeschicht nach Anspruch 23, die eine Natriumcarboxylmethyl-Zellulose umfasst.

25. Eine Gewebeschicht nach Anspruch 22, wobei das Polymer von Amid beinhaltenden Einheiten in einem zwischen 0,1 und 60 mal höheren Anteil als der des Zellulose-Derivats vorliegt.

26. Eine Gewebeschicht nach Anspruch 25, wobei das Polymer von Amid beinhaltenden Einheiten in einem höheren Anteil als der des Zellulose-Derivats vorliegt.

27. Eine Gewebeschicht nach einem der vorhergehenden Ansprüche, die eine Bruchenergie der Haut von nicht weniger als 10.000 N/m besitzt.

28. Eine Gewebeschicht nach einem der vorhergehenden Ansprüche, die außerdem einen Weichmacher umfasst.

29. Eine Gewebeschicht nach Anspruch 28, wobei der Weichmacher ein Polyalkohol ist.

30. Eine Gewebeschicht nach Anspruch 29, wobei der Weichmacher ein Glycerol ist.

31. Eine Gewebeschicht nach einem der vorhergehenden Ansprüche, wobei die Gewebeschicht außerdem ein synthetisches oder biologisches Strukturpolymer umfasst, um der Gewebeschicht Festigkeit und Elastizität zu verleihen.

32. Eine Gewebeschicht nach Anspruch 31, wobei das Strukturpolymer aus einer Gruppe, bestehend aus Polyvinylalkohol, Polyäthylenglykol, Polyacrylsäure, Polyacrylamid und ähnlichen Stoffen, ausgewählt ist.

33. Eine Gewebeschicht nach einem der vorhergehenden Ansprüche, die außerdem ein Tensid oder mehrere Tenside umfasst.

34. Eine Gewebeschicht nach Anspruch 33, die einen Weichmacher in Form eines Copolymer aus Äthylenoxid und Propylenoxid umfasst.

35. Eine Gewebeschicht nach Anspruch 1, die umfasst:
a) einen polymerisierbare und/oder vernetzbaren Stoff - von 2 bis 80 Gew.%, bevorzugt 5% bis 60% und am bevorzugsten 10% bis 30%;
b) ein bioadhesives Polymer - von 5 bis 90 Gew.%, bevorzugt 20% bis 80% und am bevorzugsten 30% bis 60%;
c) ein Strukturpolymer - von 0,01 bis 20 Gew.%, bevorzugt 1% bis 15% und am bevorzugsten 2% bis 10%;
d) ein Tensid - von 0,001% bis 10%, bevorzugt 0,01% bis 1% und am bevorzugsten 0,01% bis 0,1%;
e) einen Weichmacher - von 1% bis 70%, bevorzugt 10% bis 60% und am bevorzugsten 20% bis 40%.

36. Eine Gewebeschicht nach einem der vorhergehenden Ansprüche, die zwischen 2% und 60% Wasser nach Gewicht und vorzugsweise zwischen 5% und 30% umfasst.

37. Ein Herstellungsverfahren für eine Gewebeschicht nach einem der vorhergehenden Ansprüche, dessen Verfahren Gestaltungslösungen der folgenden Zusammensetzungen umfasst:
a) Lösung A:
i) einen polymerisierbaren und/oder vernetzbaren Stoff: 5-60%, bevorzugt 10-50% und am bevorzugsten 20-40%.
ii) ein Strukturpolymer: 0,1-30%, bevorzugt 1-20% und am bevorzugsten 3-10%.
iii) ein Tensid: 0,001-5%, bevorzugt 0,01-1% und am bevorzugsten 0,05-0,5%.
iv) einen Weichmacher: 1-80%, bevorzugt 10-60% und am bevorzugsten 15-35%.
b) Lösung B:
i) ein bioadhesives Polymer: 1-60%, bevorzugt 5-40% und am bevorzugsten 10-30%.
ii) einen Weichmacher: 1-60%, bevorzugt 5-40% und am bevorzugsten 10-30%.
Kombination von Lösung A mit Lösung B und Herbeiführung und Zulassung der In-Gang-Setzung der kombinierten Lösung.

38. Ein Verfahren nach Anspruch 37, wobei die genannte Kombination die Anordnung der Lösung A in einer Form und die Herbeiführung und Zulassung der In-Gang-Setzung durch Verdampfung, um eine erste Schicht zu gestalten und die Anordnung der Lösung B auf der ersten Schicht umfasst.

39. Eine Gewebeschicht nach Anspruch 1, die die Gestalt eines Schwammes, der maschenähnlich ist und offensichtlich Strukturoffen, wobei nur ein geringer Anteil des Gesamtvolumens der Struktur von einem festen Stoff eingenommen ist, besitzt.

40. Eine Gewebeschicht nach Anspruch 39, die umfasst:
a) einen bioadhesiven, polymerisierbaren und/oder vernetzbaren Stoff: von 1 bis 30 Gew.%, bevorzugt 5% bis 30% und am bevorzugsten 10% bis 25%;
b) Tensid - von 0,01% bis 20%, bevorzugt 0,1% bis 15% und am bevorzugsten 1% bis 15%;
c) Weichmacher - von 1% bis 50%, bevorzugt 5% bis 30% und am bevorzugsten 10% bis 25%.

41. Eine Gewebeschicht nach Anspruch 39 oder nach Anspruch 40, eine Oberfläche, die einen zusammenhängenden Überzug aus einem synthetischen oder natürlich vorkommenden polymeren Stoff besitzt.

42. Ein Herstellungsverfahren für eine Gewebeschicht nach einem der Ansprüche 39 bis 41, dessen Verfahren Gestaltungslösungen der folgenden Zusammensetzungen umfasst:
a) Lösung A:
i) einen bioadhesiven, polymerisierbaren und/oder vernetzbaren Stoff: 5-35%, bevorzugt 10-30% und am bevorzugsten 20-30%.
ii) ein Tensid: 0,01-20%, bevorzugt 0,1-15% und am bevorzugsten 1-15%.
b) Lösung B:
i) einen bioadhesiven, polymerisierbaren und/oder vernetzbaren Stoff: 1-30%, bevorzugt 5-30% und am bevorzugsten 10-25%.
ii) einen Weichmacher: 1-90%, bevorzugt 10-60% und am bevorzugsten 10-50%.
iii) ein Tensid: 0,01-20%, bevorzugt 0,1-15% und am bevorzugsten 1-15%.
Rühren der Lösungen A und B zur Schaumgestalt, Vermischen der Lösungen A und B zur Gelgestalt und Gefriertrocknen des Gels.

43. Ein Verfahren nach Anspruch 42, das außerdem eine Vernetzung des Gels umfasst.

44. Ein Verfahren nach Anspruch 43, wobei die Vernetzung durchgeführt wird, indem das Gel ionisierender Strahlung ausgesetzt wird.

45. Ein Verfahren nach einem der Ansprüche 42 bis 44, das außerdem ein Anschwellen des Gels umfasst.

46. Ein Herstellungsverfahren für eine selbstklebende, umweltverträgliche und hydratisierbare polymere Gewebeschicht in Form eines Blattes, eines Pflasters oder einer Membran, die für die Anwendung auf feuchten Oberflächen, sowohl innen als auch auf der äußeren Oberfläche des Körpers, geeignet ist, dessen Verfahren die Gestaltung einer Schaumlösung eines natürlich vorkommenden oder synthetisch polymerisierbaren und/oder vernetzbaren Stoffes, der die Wundheilung fördert, und eines synthetischen Polymers, das bioadhesive Eigenschaften besitzt und die Unterziehung der genannten Schaumlösung einer Gefriertrocknung, umfasst.

47. Ein Verfahren nach Anspruch 46, das die Schäumung einer Lösung, die alle Komponenten der Gewebeschicht beinhaltet, umfasst.

48. Ein Verfahren nach Anspruch 46, das die Gestaltung einer ersten Lösung eines natürlich vorkommenden oder synthetisch polymerisierbaren und/oder vernetzbaren Stoffes und einer zweiten Lösung eines synthetischen Polymers, das bioadhesive Eigenschaften besitzt, Aufschäumen der ersten Lösung und der zweiten Lösung und dann die Mischung der ersten und zweiten Lösung, umfasst.

49. Ein Verfahren nach einem der Ansprüche 42 bis 48, das mit einem verringerten pH-Wert durchgeführt wird.

50. Ein Verfahren nach Anspruch 49, das mit einem pH-Wert von weniger als 4,0 durchgeführt wird.

51. Ein Verfahren nach Anspruch 49, das mit einem pH-Wert von weniger als 3,0 durchgeführt wird

52. Ein Verfahren nach einem der Ansprüche 49 bis 51, das die Gestaltung einer Lösung oder mehrerer Lösungen in einem niedrigen pH-Puffer einschließt.

53. Ein Verfahren nach einem der Ansprüche 49 bis 52, das ein Anschwellen des Gels in einem niedrigen pH-Puffer einschließt.

54. Eine Gewebeschicht nach Anspruch 1, die eine Dicke von 20 -1000 µm besitzt.

55. Eine Gewebeschicht nach Anspruch 39, die eine Dicke von 0,1 bis 10 mm besitzt.

56. Die Verwendung der Gewebeschicht nach einem der Ansprüche 1 bis 36 oder 39 bis 41 in der Herstellung einer Zusammensetzung zur Prävention und Hemmung von post-chirurgischen Adhäsionen.

## Revendications

1. Une matrice polymérique auto-adhésive, biocompatible et hydratable sous forme d'une feuille, une plaque ou une pellicule adéquate pour son application pour humidifier des surfaces autant à l'intérieur que sur la surface extérieure du corps, la matrice comprenant un matériau présent dans la nature ou synthétique, polymérisable et/ou réticulable, qui favorise la cicatrisation de plaies, et un polymère synthétique ayant des propriétés bioadhésives, permettant ces propriétés, à la matrice, de s'adhérer au tissu sous-jacent au moyen d'une liaison ionique et/ou hydrogène.

2. Une matrice selon la revendication 1, qui comprend en plus un médicament, la matrice servant comme véhicule d'administration pour le médicament.

3. Une matrice selon la revendication 1, qui est libre de médicament.

4. Une matrice selon l'une quelconque des revendications précédentes, dans laquelle le composant polymérisable et/ou réticulable de la matrice est choisi parmi les polysaccharides, les polylactates, les polyalcools et les protéines.

5. Une matrice selon la revendication 4, dans laquelle le composant polymérisable et/ou réticulable de la matrice est une protéine ou un matériau protéique qui peut être réticulé en appliquant de la chaleur ou de l'énergie électromagnétique.

6. Une matrice selon la revendication 5, dans laquelle la matrice comprend de l'albumine.

7. Une matrice selon la revendication 6, dans laquelle l'albumine est de l'albumine provenant de mammifères telle que de l'albumine porcine, bovine ou humaine.

8. Une matrice selon l'une quelconque des revendications 1 à 3, dans laquelle le composant polymérisable et/ou réticulable de la matrice est un polysaccharide ou un dérivé de celui-ci.

9. Une matrice selon la revendication 8, dans laquelle le polysaccharide est un dérivé de la cellulose.

10. Une matrice selon la revendication 9, dans laquelle le dérivé de la cellulose est un éther de cellulose ou un dérivé ou un sel de celui-ci.

11. Une matrice selon la revendication 9, dans laquelle le dérivé de la cellulose est la carboxyméthylcellulose de sodium.

12. Une matrice selon l'une quelconque des revendications précédentes, dans laquelle le composant polymère bioadhésif de la matrice contient des groupements carboxyle, amide, hydroxyle, éther ou ester.

13. Une matrice selon la revendication 12, dans laquelle le polymère bioadhésif est choisi parmi le groupe formé par des acides poly(carboxyliques) et leurs dérivés, des copolymères d'acides carboxyliques et leurs dérivés et des polyalcools et leurs dérivés.

14. Une matrice selon la revendication 12, dans laquelle le polymère bioadhésif est composé d'unités structurales récurrentes contenant des groupements amide.

15. Une matrice selon la revendication 14, dans laquelle l'unité structurale récurrente est, ou contient, un groupement 1-éthylènepyrrolidin-2-one (vinylpyrrolidone).

16. Une matrice selon la revendication 14, dans laquelle le polymère est de la poly(vinylpyrrolidone).

17. Une matrice selon la revendication 12, dans laquelle le polymère bioadhésif est un copolymère d'unités contenant des amides et d'unités contenant des esters de l'acide carboxylique.

18. Une matrice selon la revendication 17, dans laquelle le copolymère est un copolymère de poly(vinylpyrrolidone)/poly(vinylacétate).

19. Une matrice selon la revendication 1, dans laquelle le polymère ayant des propriétés bioadhésives est un dérivé de la cellulose.

20. Une matrice selon la revendication 19, dans laquelle le dérivé de la cellulose est un éther de cellulose ou un dérivé ou un sel de celui-ci.

21. Une matrice selon la revendication 20, dans laquelle le dérivé de la cellulose est la carboxyméthylcellulose de sodium.

22. Une matrice selon la revendication 1, qui comprend une combinaison d'un polymère d'unités contenant des amides et un dérivé de la cellulose.

23. Une matrice selon la revendication 22, qui comprend de la poly(vinylpyrrolidone) et un dérivé de la carboxyméthylcellulose ou un sel de celui-ci.

24. Une matrice selon la revendication 23, qui comprend de la carboxyméthylcellulose de sodium.

25. Une matrice selon la revendication 22, dans laquelle le polymère d'unités contenant des amides est présent en une proportion entre 0,1 et 60 fois celle du dérivé de la cellulose.

26. Une matrice selon la revendication 25, dans laquelle le polymère d'unités contenant des amides est présent en une plus grande proportion que le dérivé de la cellulose.

27. Une matrice selon l'une quelconque des revendications précédentes, qui a une énergie de rupture par détachement non inférieure à 10.000 N/m.

28. Une matrice selon l'une quelconque des revendications précédentes, qui comprend en plus un plastifiant.

29. Une matrice selon la revendication 28, dans laquelle le plastifiant est un polyalcool.

30. Une matrice selon la revendication 29, dans laquelle le plastifiant est du glycérol.

31. Une matrice selon l'une quelconque des revendications précédentes, dans laquelle la matrice comprend en plus un polymère structural synthétique ou biologique pour donner de la force et de l'élasticité à la matrice.

32. Une matrice selon la revendication 31, dans laquelle le polymère structural est choisi parmi le groupe composé de poly(vinylalcool), poly(éthylène glycol), poly(acide acrylique), poly(acrylamide) et des matériaux semblables.

33. Une matrice selon l'une quelconque des revendications précédentes, qui comprend en plus un ou plusieurs surfactants.

34. Une matrice selon la revendication 33, qui comprend un surfactant sous forme de copolymère d'oxyde d'éthylène et d'oxyde de propylène.

35. Une matrice selon la revendication 1, qui comprend:
a) un matériau polymérisable et/ou réticulable - de 2% à 80% en poids, plus préférablement de 5% à 60%, et plus préférablement encore de 10% à 30%;
b) un(des) polymère(s) bioadhésif(s) - de 5% à 90% en poids, plus préférablement de 20% à 80%, et plus préférablement encore de 30% à 60%;
c) un polymère structural - de 0,01% à 20% en poids, plus préférablement de 1% à 15%, et plus préférablement encore de 2% à 10%;
d) un surfactant - de 0,001% à 10%, plus préférablement de 0,01% à 1%, et plus préférablement encore de 0,01% à 0,1%;
e) un plastifiant - de 1% à 70%, plus préférablement de 10% à 60% et plus préférablement encore de 20% à 40%.

36. Une matrice selon l'une quelconque des revendications précédentes, qui contient entre 2% et 60% d'eau en poids, et de préférence entre 5% et 30%.

37. Un procédé pour la fabrication d'une matrice selon l'une quelconque des revendications précédentes, ledit procédé comprenant la formation de solutions avec les compositions suivantes:
a) Solution A:
i) matériau polymérisable et/ou réticulable: 5 - 60%, plus préférablement 10 - 50% et plus préférablement encore 20 - 40%;
ii) polymère structural: 0,1 - 30%, plus préférablement 1 - 20%, et plus préférablement encore 3 - 10%.
iii) surfactant: 0,001 - 5%, plus préférablement 0,01 - 1%, et plus préférablement encore 0,05 - 0,5%.
iv) plastifiant: 1 - 80%, plus préférablement 10 - 60%, et plus préférablement encore 15 - 35%.
b) Solution B:
i) polymère(s) bioadhésif(s): 1 - 60%, plus préférablement 5 - 40%, et plus préférablement encore 10 - 30%.
ii) plastifiant: 1 - 60%, plus préférablement 5 - 40%, et plus préférablement encore 10 - 30%.
en combinant la Solution A avec la Solution B, et en provoquant ou permettant la solidification des solutions combinées.

38. Un procédé selon la revendication 37, dans lequel ladite combinaison comprend le versement de la Solution A dans un moule et provoquer ou permettre sa solidification par évaporation pour former une première couche, et le versement de la Solution B sur la première couche.

39. Une matrice selon la revendication 1, qui a la forme d'une éponge, en étant semblable à un filet, et qui est ouverte de façon évidente quant à sa structure, avec seulement une proportion minoritaire du volume total de la structure étant occupée par du matériel solide.

40. Une matrice selon la revendication 39, qui comprend:
a) un matériau bioadhésif, polymérisable et/ou réticulable - de 1% à 30% en poids, plus préférablement de 5% à 30% et plus préférablement encore de 10% à 25%;
b) un surfactant - de 0,01% à 20%, plus préférablement de 0,1% à 15% et plus préférablement encore de 1% à 15%;
c) un plastifiant - de 1% à 50%, plus préférablement de 5% à 30% et plus préférablement encore de 10% à 25%.

41. Une matrice selon la revendication 39 ou la revendication 40, possédant, une surface de celle-ci, un revêtement continu d'un matériau polymérique synthétique ou présent dans la nature.

42. Un procédé pour la fabrication d'une matrice selon l'une quelconque des revendications 39 à 41, ledit procédé comprenant la formation de solutions avec les compositions suivantes:
a) Solution A:
i) matériau bioadhésif polymérisable et/ou réticulable: 5 - 35%, plus préférablement 10 - 30% et plus préférablement encore 20 - 30%.
ii) surfactant: 0,01 - 20%, plus préférablement 0,1 - 15% et plus préférablement encore 1 - 15%.
b) Solution B:
i) matériau bioadhésif polymérisable et/ou réticulable: 1 - 30%, plus préférablement 5 - 30% et plus préférablement encore 10 - 25%.
ii) plastifiant: 1 - 90%, plus préférablement 10-60%, et plus préférablement encore 10 - 50%.
iii) surfactant: 0,01 - 20%, plus préférablement 0,1 - 15%, et plus préférablement encore 1 - 15%.
en agitant les solutions A et B pour donner lieu à des mousses, en mélangeant les solutions A et B pour donner un gel et en effectuant la lyophilisation du gel.

43. Un procédé selon la revendication 42, qui comprend en plus la réticulation du gel.

44. Un procédé selon la revendication 43, dans lequel la réticulation est réalisée en exposant le gel à un rayonnement ionisant.

45. Un procédé selon l'une quelconque des revendications 42 à 44, qui comprend en plus le gonflement du gel.

46. Un procédé pour la fabrication d'une matrice polymérique auto-adhésive, biocompatible et hydratable sous forme d'une feuille, une plaque ou une pellicule adéquate pour son application pour humidifier des surfaces autant à l'intérieur que sur la surface extérieure du corps, ledit procédé comprenant la formation d'une solution moussée d'un matériau présent dans la nature ou synthétique, polymérisable et/ou réticulable, qui favorise la cicatrisation de plaies et un polymère synthétique ayant des propriétés bioadhésives, et la lyophilisation de ladite solution moussée.

47. Un procédé selon la revendication 46, qui comprend le moussage d'une solution contenant tous les composants de la matrice.

48. Un procédé selon la revendication 46, qui comprend la formation d'une première solution du matériau présent dans la nature ou synthétique polymérisable et/ou réticulable, et une seconde solution du polymère synthétique ayant des propriétés bioadhésives, le moussage de la première solution et la seconde solution, et le mélange, par la suite, de la première et la seconde solution.

49. Un procédé selon l'une quelconque des revendications 42 à 48, qui est réalisé à un pH réduit.

50. Un procédé selon la revendication 49, qui est réalisé à un pH en dessous de 4,0.

51. Un procédé selon la revendication 49, qui est réalisé à un pH en dessous de 3,0.

52. Un procédé selon l'une quelconque des revendications 49 à 51, qui implique la formation d'une ou plusieurs solutions dans un tampon à bas pH.

53. Un procédé selon l'une quelconque des revendications 49 à 52, qui implique le gonflement du gel dans un tampon à bas pH.

54. Une matrice selon la revendication 1, qui a une épaisseur de 20 - 1000 µm.

55. Une matrice selon la revendication 39, qui a une épaisseur de 0,1 à 10 mm.

56. L'utilisation d'une matrice selon l'une quelconque des revendications 1 à 36 ou 39 à 41 dans la fabrication d'une composition pour prévenir ou inhiber les adhérences post-chirurgicales.
